# EUROPÄISCHE PATENTANMELDUNG

(11) **EP 3 327 457 A1**
(43) Veröffentlichungstag der Anmeldung: **30.05.2018**
(21) Anmeldenummer: 16200280.2
(22) Anmeldetag: 23.11.2016
(51) Int. Cl.: G01R 33/48, A61B 6/00, A61N 5/10, G01R 33/3815

(54) **MEDIZINISCHES BILDGEBUNGSSYSTEM UMFASSEND EINE MAGNETEINHEIT UND EINE STRAHLUNGSEINHEIT**

(71) Anmelder: Siemens Healthcare GmbH, 91052 Erlangen (DE)
(72) Erfinder: Leghissa, Martino, 91369 Wiesenthau (DE); Fahrig, Rebecca, 91096 Möhrendorf (DE)

(57) **Zusammenfassung**

Die Erfindung betrifft ein medizinisches Bildgebungssystem, umfassend eine Magneteinheit (20) ausgebildet zur Magnetresonanzbildgebung eines Untersuchungsobjekts sowie eine erste Strahlungseinheit (30) ausgebildet zum Bestrahlen des Untersuchungsobjekts, wobei die Magneteinheit einen Hauptmagneten sowie ein Gehäuse aufweist, wobei der Hauptmagnet innerhalb des Gehäuses angeordnet ist und wobei der Hauptmagnet Spulenelemente und wenigstens einen Spulenträger aufweist. Weiterhin definiert die Magneteinheit derart eine Untersuchungsöffnung (90) entlang einer Untersuchungsachse definiert, dass die Magneteinheit die Untersuchungsöffnung umschließt. Weiterhin weist die Magneteinheit einen ersten Bereich auf, der für radial zur Untersuchungsachse ausgesendete Strahlung der ersten Strahlungseinheit transparent ist. Weiterhin ist die erste Strahlungseinheit an der von der Untersuchungsöffnung abgewandten Seite der Magneteinheit angeordnet und dazu ausgebildet, Strahlung durch den ersten Bereich der Magneteinheit in Richtung der Untersuchungsöffnung zu senden. Weiterhin ist erste Strahlungseinheit weiterhin dazu ausgebildet, um die Untersuchungsöffnung zu rotieren.

## Beschreibung

Die Magnetresonanztomographie (kurz MR) ist ein bildgebendes Verfahren, das sich durch besonders hohen Weichteilkontrast auszeichnet. Sie kann daher insbesondere bei der Diagnostik von Tumoren und in der Angiographie eingesetzt werden.

In diesen Anwendungsgebieten ist es weiterhin bekannt, Strahlungseinheiten wie Röntgenquellen oder Teilchenstrahlungsquellen einzusetzen. Bei Teilchenstrahlung kann es sich dabei insbesondere um Elektronenstrahlung oder um Hadronenstrahlung handeln. Diese Strahlungseinheiten können einerseits zusammen mit einem geeigneten Detektor zur Bildgebung, andererseits zur Manipulation von Gewebe eingesetzt werden.

Für viele medizinische Diagnostikanwendung ist es wünschenswert, sowohl MR-Bilddaten als auch aus Strahlungseinheiten gewonnene Bilddaten zu verwenden. Weiterhin ist es bei der Manipulation von Gewebe mittels Teilchenstrahlung wünschenswert, die Position und andere Charakteristika des bestrahlten Gewebes mittels MR-Bildgebung zu überwachen.

Es ist bekannt, eine MR-Bildgebung und Bestrahlung zeitlich versetzt mittels eines MR-Geräts und mittels eines vom MR-Gerät separaten Strahlungsgeräts durchzuführen. Hierbei muss der Patient aber zwischen den beiden unterschiedlichen Geräten transportiert werden oder sogar umgelagert werden. Weiterhin kann sich die Anatomie des Patienten aufgrund des Zeitraums zwischen den beiden Vorgängen ändern, z.B. durch Atmung oder Stoffwechselvorgänge. Dies erschwert eine Kombination der MR-Bildgebung mit der Bestrahlung.

Aus der Druckschrift US 2015/0247907 A1 ist es bekannt, eine Röntgenquelle sowie einen Röntgendetektor in der Untersuchungsöffnung eines Magnetresonanztomographen zu betreiben. Hierfür müssen aber sowohl die Röntgenquelle als auch der Röntgendetektor dafür ausgelegt sein, in starken Magnetfeldern betrieben zu werden.

Weiterhin ist aus der Veröffentlichung GANGULY, Arundhuti et al.: "Truly Hybrid X-Ray/MR Imaging: Toward a Streamlined Clinical System", in: Academic Radiology (12) 2005, Seiten 1167 - 1177, bekannt, ein MR-Gerät zu verwenden, der mehrere Magneteinheiten aufweist, und die Strahlungseinheit so auszubilden, dass der Strahlengang zwischen den unterschiedlichen Magneteinheiten hindurch verläuft. Hierdurch wird die Strahlungseinheit zwar nicht vom starken Magnetfeld in der Untersuchungsöffnung des MR-Geräts beeinflusst, durch die mehreren Magneteinheiten ist es aber schwierig, ein für die MR-Bildgebung notwendiges homogenes Magnetfeld in der Untersuchungsöffnung zu erreichen.

Es ist daher die Aufgabe der vorliegenden Erfindung, eine effiziente Möglichkeit für eine gleichzeitige MR-Bildgebung und Strahlungsexposition des Untersuchungsobjekts bereitzustellen.

Die Aufgabe wird durch das medizinische Bildgebungssystem nach dem unabhängigen Anspruch gelöst. Vorteilhafte Ausgestaltungen sind in den Unteransprüchen beschrieben.

Die Erfindung betrifft ein medizinisches Bildgebungssystem, umfassend eine Magneteinheit ausgebildet zur Magnetresonanzbildgebung eines Untersuchungsobjekts sowie eine erste Strahlungseinheit ausgebildet zum Bestrahlen des Untersuchungsobjekts, wobei die Magneteinheit einen Hauptmagneten sowie ein Gehäuse aufweist, wobei der Hauptmagnet innerhalb des Gehäuses angeordnet ist und wobei der Hauptmagnet Spulenelemente und wenigstens einen Spulenträger aufweist. Weiterhin definiert die Magneteinheit derart eine Untersuchungsöffnung entlang einer Untersuchungsachse, dass die Magneteinheit die Untersuchungsöffnung umschließt. Weiterhin weist die Magneteinheit einen ersten Bereich auf, der für radial zur Untersuchungsachse ausgesendete Strahlung der ersten Strahlungseinheit transparent ist. Weiterhin ist die erste Strahlungseinheit auf der von der Untersuchungsöffnung abgewandten Seite der Magneteinheit angeordnet und dazu ausgebildet, Strahlung durch den ersten Bereich der Magneteinheit in Richtung der Untersuchungsöffnung zu senden. Weiterhin ist erste Strahlungseinheit weiterhin dazu ausgebildet, um die Untersuchungsöffnung zu rotieren.

Der erste Bereich der Magneteinheit ist insbesondere derart für Strahlung der ersten Strahlungseinheit transparent, dass die Intensität von radial zur Untersuchungsachse und durch den ersten Bereich verlaufende Strahlung der ersten Strahlungseinheit in einem geringeren Maße abgeschwächt wird als die Intensität von radial zur Untersuchungsachse und nicht durch den ersten Bereich verlaufende Strahlung der ersten Strahlungseinheit. Insbesondere beträgt die Abschwächung der Intensität der den ersten Bereich durchlaufenden Strahlung nach Durchlauf der Magneteinheit weniger als die Hälfte oder weniger als 10% oder weniger als 1% der Abschwächung der Intensität der nicht den ersten Bereich durchlaufenden Strahlung nach Durchlauf der Magneteinheit. Die Untersuchungsöffnung ist insbesondere zur Aufnahme des Untersuchungsobjekts ausgebildet. Die erste Strahlungseinheit kann insbesondere außerhalb der Magneteinheit ausgebildet sein. Das medizinische Bildgebungssystem kann insbesondere genau eine Magneteinheit umfassen.

Die Erfinder haben erkannt, dass durch die Anordnung der Strahlungseinheit außerhalb der Magneteinheit diese nicht durch das starke Hauptmagnetfeld in der Untersuchungsöffnung während der MR-Bildgebung beeinflusst wird. Es können daher kostengünstige Strahlungseinheiten verwendet werden, die nicht dafür ausgelegt sind, in starken Magnetfeldern betrieben zu werden.

Als weiterer Vorteil einer Anordnung der ersten Strahlungseinheit außerhalb der Magneteinheit im Vergleich zur Anordnung in der Untersuchungsöffnung ergibt sich, dass außerhalb der Magneteinheit mehr Platz für die erste Strahlungseinheit zur Verfügung steht, und daher effizientere und/oder stärkere erste Strahlungseinheiten verwendet werden können. Weiterhin haben die Erfinder erkannt, dass durch eine Anordnung außerhalb des Magnetfeldes die erste Strahlungseinheit auch sehr einfach für Wartungsmaßnahmen zugänglich ist.

Weiterhin haben die Erfinder erkannt, dass es durch den für die Strahlung der ersten Strahlungseinheit transparente Bereich der Magneteinheit nicht notwendig ist, mehrere separierte Magneteinheiten zu verwenden. Dadurch lässt sich ein homogeneres Magnetfeld und somit eine genauere MR-Bildgebung erreichen als mit mehreren separierten Magneteinheiten.

Nach einem weiteren möglichen Aspekt der Erfindung weist die Magneteinheit weiterhin einen ersten Austrittsbereich auf, der für radial zur Untersuchungsachse durch den ersten Bereich und das Untersuchungsobjekt gesendete Strahlung der ersten Strahlungseinheit transparent ist, und wobei der erste Austrittsbereich nicht mit dem ersten Bereich überlappt. Die Erfinder haben erkannt, dass durch eine zusätzliche ersten Austrittsbereich zum einen ein Strahlungsdetektor auch außerhalb der Magneteinheit angeordnet werden kann. Weiterhin kann durch einen ersten Austrittsbereich die ungestreute Strahlung der ersten Strahlungseinheit aus der Magneteinheit ausgeleitet werden, ohne mit der Magneteinheit zu interagieren und diese zu beschädigen.

Nach einem weiteren Aspekt der Erfindung sind die Spulenelemente des Hauptmagneten und der wenigstens eine Spulenträger außerhalb des ersten Bereichs der Magneteinheit angeordnet. Die Erfinder haben erkannt, dass durch diese Geometrie der Spulenelemente und des Spulenträgers des Hauptmagneten eine Transparenz für die Strahlung der Strahlungseinheit besonders effizient und kostengünstig erreicht werden kann, da für die Spulenelemente des Hauptmagneten und für den wenigstens einen Spulenträger keine speziellen Materialien verwendet werden müssen und kein Intensitätsverlust der Strahlung bei der Durchleuchtung der Spulenelemente des Hauptmagneten oder des wenigstens einen Spulenträgers auftritt.

Nach einem weiteren Aspekt der Erfindung weist die Magneteinheit im ersten Bereich wenigstens ein inneres Fenster und wenigstens ein äußeres Fenster im Gehäuse auf, wobei das innere Fenster und das äußere Fenster für die von der ersten Strahlungseinheit ausgesendete Strahlung transparent sind. Hierbei ist das innere Fenster auf der von der Untersuchungsöffnung zugewandten Seite der Magneteinheit, insbesondere als Teil des Gehäuses, angeordnet. Weiterhin ist hierbei das äußere Fenster auf der von der Untersuchungsöffnung abgewandten Seite der Magneteinheit, insbesondere als Teil des Gehäuses, angeordnet. Die Erfinder haben erkannt, dass durch die Verwendung wenigstens zweier Fenster die Struktur und damit die Stabilität der Magneteinheit erhalten werden kann, und gleichzeitig eine Transparenz des ersten Bereichs der Magneteinheit erreicht werden kann.

Das innere Fenster und das äußere Fenster sind insbesondere derart transparent, dass das Material des inneren und des äußeren Fensters für die Strahlung der ersten Strahlungseinheit transparenter ist als das Material des Gehäuses. Insbesondere beträgt die Abschwächung der Intensität von Strahlung bei Durchlauf des Fensters weniger als die Hälfte oder weniger als 10% oder weniger als 1% der Abschwächung der Intensität bei Durchlauf des Gehäuses.

Nach einem weiteren Aspekt der Erfindung ist der erste Bereich als Trichter in der Magneteinheit ausgebildet, der sich radial zur Untersuchungsachse erstreckt und der für die von der ersten Strahlungseinheit radial zur Untersuchungsachse ausgesendete Strahlung durchdringbar ist. Bei einem Trichter handelt es sich insbesondere um eine durchgehende Öffnung der Magneteinheit. Die Erfinder haben erkannt, dass durch die Verwendung eines Trichters die von der Strahlungseinheit durch die Magneteinheit gesendete Strahlung nur sehr wenig abgeschwächt werden.

Nach einem weiteren Aspekt der Erfindung kann der Trichter insbesondere durch das Gehäuse der Magneteinheit ausgebildet werden, insbesondere können die Seitenwände des Trichters durch das Gehäuse ausgebildet werden. Die Erfinder haben erkannt, dass es die Ausbildung des Trichters durch das Gehäuse ermöglicht, die Magneteinheit besonders stabil auszubilden, und insbesondere ein geschlossenes Kühlsystem möglichst einfach und kostengünstig auszubilden.

Nach einem weiteren Aspekt der Erfindung kann der Trichter insbesondere mit einem für die Strahlung der ersten Strahlungseinheit transparentem Material gefüllt sein. Die Erfinder haben erkannt, dass sich durch die Füllung mit einem strahlungsdurchlässigen Material die Stabilität der Magneteinheit erhöhen lässt.

Nach einem weiteren möglichen Aspekt der Erfindung weist das medizinische Bildgebungssystem weiterhin einen ersten Strahlungsdetektor auf, wobei der erste Strahlungsdetektor dazu ausgebildet ist, von der ersten Strahlungseinheit durch den ersten Bereich der Magneteinheit gesendete Strahlung zu detektieren, und wobei der erste Strahlungsdetektor auf der von der ersten Strahlungseinheit abgewandten Seite des Untersuchungsobjekts angeordnet ist. Bei einem ersten Strahlungsdetektor kann es sich insbesondere um einen ersten Röntgendetektor, um einen Gammadetektor und/oder um einen Teilchendetektor handeln. Die Erfinder haben erkannt, dass eine Detektion der Strahlung genutzt werden kann, um die Strahlungsdosis zu messen, denen das Untersuchungsobjekt durch eine Bestrahlung mit der Strahlung der ersten Strahlungseinheit ausgesetzt ist, und damit die Strahlungsdosis zu minimieren.

Nach einem weiteren Aspekt der Erfindung ist die erste Strahlungseinheit eine Teilchenquelle, die dazu ausgebildet ist, Teilchenstrahlung zu erzeugen. Die Erfinder haben erkannt, dass durch ein Bestrahlen mittels Teilchenstrahlung besonders viel Energie in einem Gewebe deponiert werden kann, und die Bestrahlung daher besonders effektiv ist.

Nach einem weiteren möglichen Aspekt der Erfindung ist die Teilchenquelle dazu ausgebildet, Elektronen- und/oder Hadronenstrahlung zu erzeugen. Die Erfinder haben erkannt, dass Elektronen- und/oder Hadronenstrahlung besonders einfach und kostengünstig mit einer Teilchenstrahlungsquelle erzeugt werden können.

Nach einem weiteren Aspekt der Erfindung ist die erste Strahlungseinheit eine erste Röntgenquelle. Weiterhin weißt das medizinische Bildgebungssystem einen ersten Röntgendetektor auf, wobei der erste Röntgendetektor auf der von der ersten Röntgenquelle abgewandten Seite des Untersuchungsobjekts angeordnet ist, und wobei die erste Röntgenquelle und der erste Röntgendetektor zur Röntgenbildgebung des Untersuchungsobjekts ausgebildet sind. Die Erfinder haben erkannt, dass mittels von einer erste Röntgenquelle emittierter und mittels eines ersten Röntgendetektors empfangener Röntgenstrahlung eine Bildgebung möglich ist, die sich gut mit der MR-Bildgebung ergänzt, denn die MR-Bildgebung hat einen guten Weichteilkontrast, und die Röntgenbildgebung kann gut Knochenstrukturen und Kontrastmittel darstellen.

Die Erfinder haben weiterhin erkannt, dass sich durch eine Anordnung der ersten Röntgenquelle außerhalb der Magneteinheit ein größerer Abstand und ein kleinerer Vergrößerungsfaktor der Röntgenprojektionen ergeben als durch eine Anordnung in der Untersuchungsöffnung. Dadurch verringert sich die von der Oberfläche des Untersuchungsobjekts, insbesondere der Haut eines Patienten absorbierte Strahlungsdosis, sowie die Verschleierung der Röntgenprojektionen.

Nach einem weiteren Aspekt der Erfindung kann der erste Röntgendetektor im Hauptmagnetfeld des Hauptmagneten angeordnet sein. Die Erfinder haben erkannt, dass ein erste Röntgendetektor einerseits deutlich unempfindlicher gegen hohe Magnetfelder als eine erste Röntgenquelle ist, andererseits durch die Anordnung im Hauptmagnetfeld die Röntgenstrahlung nur einmal den Hauptmagneten passieren muss, und kein mit einer Intensitätsschwächung verknüpftes zweites Mal. Daher muss bei dieser Anordnung auch kein Austrittsbereich der Magneteinheit für Röntgenstrahlung transparent sein.

Nach einem weiteren Aspekt der Erfindung kann der erste Röntgendetektor gekrümmt ausgebildet sein. Die Erfinder haben erkannt, dass bei einer gekrümmten Ausbildung der in der Untersuchungsöffnung für Untersuchungsobjekte verbleibende Platz größer ist, und/oder ein größerer erster Röntgendetektor verwendet werden kann.

Nach einem weiteren Aspekt der Erfindung kann die Magneteinheit einen ersten Austrittsbereich aufweisen, weiterhin ist der erste Röntgendetektor außerhalb der Magneteinheit auf der von der Untersuchungsöffnung abgewandten Seite der Magneteinheit derart vor dem ersten Austrittsbereich angeordnet ist, dass der erste Röntgendetektor von der ersten Röntgenquelle durch den ersten Bereich ausgesandte Röntgenstrahlung empfangen kann. Die Erfinder haben erkannt, dass durch die Anordnung des ersten Röntgendetektors außerhalb der Magneteinheit die Untersuchungsöffnung möglichst groß ausgebildet werden kann.

Nach einem weiteren Aspekt der Erfindung ist der erste Röntgendetektor dazu ausgebildet, simultan mit der ersten Röntgenquelle um die Untersuchungsöffnung zu rotieren. Die Erfinder haben erkannt, dass durch eine simultane Rotation die relative Position zwischen der ersten Röntgenquelle und dem erstem Röntgendetektor konstant bleibt, und die Röntgenbildgebung nicht an eine veränderte relative Position angepasst werden muss. Weiterhin ist es durch die simultane Rotation möglich, nicht einen großen, nicht-rotierbaren ersten Röntgendetektor, sondern einen möglichst kleinen und damit kostengünstigen ersten Röntgendetektor einzusetzen.

Nach einem weiteren Aspekt der Erfindung ist die Magneteinheit dazu ausgebildet, um die Untersuchungsöffnung zu rotieren. Die Erfinder haben erkannt, dass hierdurch der erste Bereich der Magneteinheit in verschiedene Ausrichtungen rotiert werden kann, und die Bestrahlung aus unterschiedlichen Richtungen durch immer nur einen transparenten Bereich der Magneteinheit erfolgen kann. Damit muss der transparente Bereich der Magneteinheit nur so groß wie der Strahlengang der Strahlungseinheit sein. Durch einen möglichst kleinen ersten Bereich vergrößert sich die Stabilität der Magneteinheit, weiterhin können die Spulenelemente des Hauptmagneten und die Spulenträger des Hauptmagneten in einem größeren Volumen angeordnet werden, dies vereinfacht die Erzeugung eines homogenen Magnetfeldes. Weiterhin vereinfacht ein möglichst kleiner erster Bereich die Anordnung einer Gradientenspuleneinheit und einer Hochfrequenzantenneneinheit außerhalb des ersten Bereichs und verbessert damit die Güte des Gradientenfeldes und des Hochfrequenzfeldes.

Nach einem weiteren Aspekt der Erfindung sind die erste Strahlungseinheit und die Magneteinheit dazu ausgebildet, simultan um die Untersuchungsöffnung zu rotieren. Insbesondere kann die erste Strahlungseinheit mit der Magneteinheit verbunden sein. Insbesondere kann die erste Strahlungseinheit derart mit der Magneteinheit verbunden sein, dass die von der ersten Strahlungseinheit ausgesendete Strahlung durch den ersten transparenten Bereich der Magneteinheit verlaufen. Die Erfinder haben erkannt, dass durch simultane Rotation der ersten Strahlungseinheit mit der Magneteinheit keine separate, zeitintensive Ausrichtung und/oder Platzierung der ersten Strahlungseinheit während der Untersuchung notwendig ist. Weiterhin kann der erste Bereich der Magneteinheit möglichst klein gewählt werden. Die Erfinder haben weiterhin erkannt, dass in dieser Anordnung das Magnetfeld der Hauptmagneteinheit außerhalb der Untersuchungsöffnung, welches die erste Strahlungseinheit durchdringt, nicht von der Orientierung der ersten Strahlungseinheit abhängt. Dadurch können in der ersten Strahlungseinheit Maßnahmen zum Ausgleich des Magnetfeldes getroffen werden, die nicht von der Orientierung der ersten Strahlungseinheit abhängen.

Nach einem weiteren Aspekt der Erfindung ist die Magneteinheit dazu ausgelegt, die Spulenelemente des Hauptmagnets mittels Wärmeleitung zu kühlen. Die Erfinder haben erkannt, dass eine Kühlung mittels Wärmeleitung kostengünstiger ist als eine konvektionsbasierte Kühlung mittels Immersion in ein Kühlmittel, denn für eine Kühlung mittels Wärmeleitung wird weniger Kühlmittel benötigt. Die Erfinder haben weiterhin erkannt, dass bei einer Kühlung mittels Wärmeleitung die Kühlleistung nicht oder nur weniger als bei einer Kühlung mittels Immersion von der Ausrichtung der Magneteinheit beeinflusst wird. Dies erlaubt eine effiziente Kühlung auch bei einer Magneteinheit, die zum Rotieren ausgelegt ist.

Nach einem weiteren Aspekt der Erfindung wird die Abwärme der Spulenelemente des Hauptmagneten durch Röhrenleitungen umfassend ein zirkulierendes Kühlmittel abgeleitet. Die Erfinder haben erkannt, dass mittels der Röhrenleitungen eine besonders effektive Kühlung möglich ist. Weiterhin haben die Erfinder erkannt, dass der erste Bereich besonders transparent für die Strahlung der ersten Strahlungseinheit ausgebildet werden kann, wenn die Röhrenleitungen außerhalb des ersten Bereichs der Magneteinheit angeordnet werden.

Nach einem weiteren Aspekt der Erfindung sind die Spulenelemente des Hauptmagneten aus elektrisch supraleitendem Material ausgebildet, wobei die kritische Temperatur des supraleitenden Materials höher ist als der Siedepunkt von Helium. Die Erfinder haben erkannt, dass durch eine kritische Temperatur, die über dem Siedepunkt von flüssigem Helium liegt, andere Kühlmittel als flüssiges Helium und/oder Kühlverfahren als eine Immersion in flüssiges Helium verwendet werden können, insbesondere eine Kühlung mittels Wärmeleitung. Diese Kühlung ist daher kostengünstiger und weiterhin besonders vorteilhaft bei einer zum Rotieren ausgebildeten Magneteinheit, weiterhin ist diese Kühlung besonders vorteilhaft, um den ersten Bereich der Magneteinheit transparent für die Strahlung der ersten Strahlungseinheit auszubilden.

Nach einem weiteren möglichen Aspekt der Erfindung ist das elektrisch leitende Material der Spulenelemente des Hauptmagneten Magnesiumdiborid. Die Erfinder haben erkannt, dass Magnesiumdiborid ein metallischer Supraleiter mit einer besonders hohen kritischen Temperatur ist. Damit können Maßnahmen zur Kühlung der Spulenelemente verwendet werden, die besonders kostengünstige sowie transparent für die von der Strahlungseinheit emittierte Strahlung sind.

Nach einem weiteren Aspekt der Erfindung umfasst dass medizinisches Bildgebungssystem weiterhin eine zweite Strahlungseinheit, wobei die zweite Strahlungseinheit auf der von der Untersuchungsöffnung abgewandten Seite der Magneteinheit angeordnet ist. Weiterhin weist die Magneteinheit einen zweiten Bereich auf, der für radial zur Untersuchungsachse ausgesendete Strahlung der zweiten Strahlungseinheit transparent ist. Weiterhin ist die zweite Strahlungseinheit dazu ausgebildet, Strahlung durch den zweiten Bereich der Magneteinheit in Richtung der Untersuchungsöffnung zu senden, weiterhin ist die zweite Strahlungseinheit dazu ausgebildet ist, um die Untersuchungsöffnung zu rotieren. Insbesondere kann die zweite Strahleneinheit außerhalb der Magneteinheit angeordnet sein. Die Erfinder haben erkannt, dass durch eine zusätzlich zu einer ersten Röntgenquelle vorhandene zweite Strahlungseinheit basierend auf der MR-Bildgebung und der Röntgenbildgebung mittels der ersten Röntgenquelle mit der zweiten Strahlungseinheit bestrahlt werden kann. Hierdurch können die sich ergänzenden Bildinformationen der MR-Bildgebung und der ersten Röntgenbildgebung verwendet werden. Hierdurch kann die zweite Strahlungseinheit besonders effizient eingesetzt werden.

Der zweite Bereich der Magneteinheit ist insbesondere derart für Strahlung der zweiten Strahlungseinheit transparent, dass die Intensität von radial zur Untersuchungsachse und durch den zweiten Bereich verlaufende Strahlung der zweiten Strahlungseinheit in einem geringeren Maße abgeschwächt wird als die Intensität von radial zur Untersuchungsachse und nicht durch den ersten Bereich und nicht durch den zweiten Bereich verlaufende Strahlung der zweiten Strahlungseinheit. Insbesondere beträgt die Abschwächung der Intensität der den zweiten Bereich durchlaufenden Strahlung bei Durchlauf weniger als die Hälfte oder weniger als 10% oder weniger als 1% der Abschwächung der Intensität der nicht durch den ersten und nicht durch den zweiten Bereich laufenden Strahlung.

Nach einem weiteren Aspekt er Erfindung ist die zweite Strahlungseinheit eine zweite Röntgenquelle, weiterhin weist das medizinische Bildgebungssystem einen zweiten Röntgendetektor auf, wobei der zweite Röntgendetektor auf der von der zweiten Röntgenquelle abgewandten Seite des Untersuchungsobjekts angeordnet ist. Weiterhin ist der zweite Röntgendetektor dazu ausgebildet, simultan mit der zweiten Röntgenquelle um die Untersuchungsöffnung zu rotieren. Weiterhin sind die zweite Röntgenquelle und der zweite Röntgendetektor zur Röntgenbildgebung des Untersuchungsobjekts ausgebildet Die Erfinder haben erkannt, dass es mit einer solchen Anordnung möglich ist, gleichzeitig zwei Röntgenprojektionen aus zwei unterschiedlichen Richtungen aufzunehmen, ohne eine Rotation der Röntgenquellen oder eine Rotation der Magneteinheit durchführen zu müssen. Hiermit ist es möglich, einen dreidimensionalen Röntgenbilddatensatz ohne Rotation der Röntgenquellen oder eine Rotation der Magneteinheit zu rekonstruieren.

Nach einem weiteren möglichen Aspekt der Erfindung weist die Magneteinheit weiterhin einen zweiten Austrittsbereich auf, der für radial zur Untersuchungsachse durch den zweiten Bereich und das Untersuchungsobjekt gesendete Strahlung der zweiten Strahlungseinheit transparent ist, wobei der zweite Austrittsbereich nicht mit dem zweiten Bereich überlappt. Die Erfinder haben erkannt, dass durch eine zusätzliche zweiten Austrittsbereich zum einen ein Strahlungsdetektor auch außerhalb der Magneteinheit angeordnet werden kann. Weiterhin kann durch einen zweiten Austrittsbereich die ungestreute Strahlung der zweiten Strahlungseinheit aus der Magneteinheit ausgeleitet werden, ohne mit der Magneteinheit zu interagieren und diese zu beschädigen.

Nach einem weiteren Aspekt der Erfindung kann der zweite Röntgendetektor alle weiteren Merkmale des ersten Röntgendetektors aufweisen. Alle Vorteile, die einer Ausführungsform des ersten Röntgendetektors zugeordnet werden, können auch der entsprechenden Ausführungsform des zweiten Röntgendetektors zugeordnet werden.

Nach einem weiteren Aspekt der Erfindung können der zweite Bereich der Magneteinheit, der erste Austrittsbereich und/oder der zweite Austrittsbereich alle weiteren Merkmale des ersten Bereichs der Magneteinheit aufweisen. Alle Vorteile, die einer Ausführungsform des ersten Bereichs der Magneteinheit zugeordnet werden, können auch den entsprechenden Ausführungsformen des zweiten Bereichs, des ersten Austrittsbereich und/oder des zweite Austrittsbereich zugeordnet werden.

Nach einem weiteren Aspekt der Erfindung schließt die Verbindungslinie von erster Röntgenquelle und erstem Röntgendetektor mit der Verbindungslinie von zweiter Röntgenquelle und zweitem Röntgendetektor einen Winkel zwischen 60 und 120 Grad oder zwischen 80 und 100 Grad oder zwischen 85 und 95 Grad ein. Die Erfinder haben erkannt, dass der Winkel zwischen den Verbindungslinien dem Winkel zwischen den Projektionsrichtungen der mit den beiden Röntgenquellen und den beiden Röntgendetektoren aufgenommen Röntgenprojektionen entspricht. Mit einem derartigen Winkel zwischen den Röntgenprojektionen können besonders effizient dreidimensionale Röntgendaten aus den zweidimensionalen Röntgenprojektionen rekonstruiert werden.

Eine Magneteinheit kann insbesondere zylinderförmig, ringförmig und/oder torusförmig ausgebildet sein. Weiterhin weist die Magneteinheit eine der Untersuchungsöffnung zugewandte Seite auf, die als innere Seite bezeichnet wird, weiterhin weist die Magneteinheit eine der Untersuchungsöffnung abgewandte Seite auf, die als äußere Seite bezeichnet wird. Die Magneteinheit umschließt die Untersuchungsöffnung derart, dass sie die Untersuchungsöffnung entlang eines Umlaufs um die Vorzugsrichtung umschließt, es die Untersuchungsöffnung ist also insbesondere an zwei Enden offen. Die Magneteinheit umschließt eine Untersuchungsöffnung auch, wenn sie konstruktionsbedingte Aussparungen aufweist.

Eine Strahlungseinheit sendet elektromagnetische Strahlung oder Teilchenstrahlung aus. Bei elektromagnetischer Strahlung kann es sich insbesondere um Röntgenstrahlung oder um Gammastrahlung handeln. Als Röntgenstrahlung bezeichnet man insbesondere elektromagnetische Strahlung mit einer Wellenlänge zwischen 1 pm und 500 pm, insbesondere zwischen 5 pm und 250 pm, insbesondere zwischen 5 pm und 60 pm. Als Gammastrahlung bezeichnet man insbesondere elektromagnetische Strahlung mit einer Wellenlänge unter 5 pm, insbesondere unter 1 pm, unabhängig von der Erzeugung der Strahlung. Das Spektrum von sowohl Röntgenstrahlung als auch Gammastrahlung kann monochromatisch oder polychromatisch sein. Teilchenstrahlung entspricht insbesondere einem Strom von Teilchen in eine gemeinsame Richtung. Bei Teilchen kann es sich insbesondere um Leptonen oder Baryonen handeln. Bei Baryonen kann es sich insbesondere um Protonen oder Neutronen handeln. Bei Leptonen kann es sich insbesondere um Elektronen, Positronen oder Myonen handeln.

Ein erster Bereich, ein zweiter Bereich und/oder ein Austrittsbereich der Magneteinheit sind insbesondere für die Strahlung der ersten Strahlungseinheit transparent, wenn die Intensität der Strahlung nach dem Durchgang durch den ersten Bereich, den zweiten Bereich und/oder den Austrittsbereich mindestens 10%, insbesondere mindestens 50%, insbesondere mindestens 90%, insbesondere mindestens 95%, insbesondere mindestens 99%, insbesondere mindestens 99,9% der Intensität der Strahlung vor dem Durchgang durch den ersten Bereich, den zweiten Bereich und/oder den Austrittsbereich beträgt. Die Magneteinheit ist insbesondere für radial zur Untersuchungsachse und durch den ersten Bereich, den zweiten Bereich und/oder den Austrittsbereich verlaufende Strahlung der ersten Strahlungseinheit transparenter als für radial zur Untersuchungsachse und nicht durch den ersten Bereich, den zweiten Bereich und/oder den Austrittsbereich verlaufende Strahlung der ersten Strahlungseinheit, falls die Abschwächung der Intensität der radial zur Untersuchungsachse und nicht durch den ersten Bereich, den zweiten Bereich und/oder den Austrittsbereich verlaufende Strahlung um mehr als einen Faktor 2, insbesondere um mehr als einen Faktor 5, insbesondere um mehr als einen Faktor 10, insbesondere um mehr als einen Faktor 50 größer ist als die Abschwächung der Intensität der radial zur Untersuchungsachse und durch den ersten Bereich, den zweiten Bereich und/oder den Austrittsbereich verlaufende Strahlung. Hierbei bezeichnet die Intensität die Energie der Strahlung pro Flächeneinheit und pro Zeiteinheit. Bei einer monochromatischen elektromagnetischen Strahlung ist die Intensität der Strahlung insbesondere proportional zur quadratischen Amplitude des variablen elektrischen Feldes. Bei einer Teilchenstrahlung ist die Intensität der Strahlung insbesondere proportional zur Energie der Teilchen sowie zur Zahl der Teilchen pro Zeiteinheit.

Eine erste Einheit und eine zweite Einheit rotieren insbesondere simultan um eine Achse oder einen Bereich, wenn sie mit der gleichen Winkelgeschwindigkeit rotieren um die Achse oder den Bereich rotieren. Insbesondere bleibt also auch der Winkel zwischen der ersten Verbindungslinie der ersten Einheit mit der Achse oder dem Bereich und der zweiten Verbindungslinie der zweiten Einheit mit der Achse oder dem Bereich konstant.

Die Kühlung der Spulenelemente eines Magneten kann durch Wärmeleitung, Wärmekonvektion und/oder Wärmestrahlung erfolgen. Die Spulenelemente des Hauptmagneten eines MR-Geräts sind beispielsweise zur Kühlung mittels Wärmekonvektion ausgebildet, indem sie in flüssigem Helium gelagert werden. Weiterhin können die Spulenelemente des Hauptmagneten eines MR-Geräts zur Kühlung mittels Wärmeleitung ausgelegt sein.

Als Supraleiter werden Materialien bezeichnet, deren elektrischer Widerstand beim Unterschreiten einer kritischen Temperatur (ein anderer Fachbegriff ist Sprungtemperatur) auf null fällt. Supraleitende Materialien werden insbesondere in Spulen und Spulenelementen zur Erzeugung von starken Magnetfeldern genutzt.

Eine erste Einheit, die auf der von einer zweiten Einheit abgewandten Seite einer dritten Einheit angeordnet ist, muss nicht Teil der dritten Einheit sein oder von der dritten Einheit umfasst werden. Eine erste Einheit ist in diesem Fall insbesondere von der dritten Einheit aus gesehen hinter der zweiten Einheit angeordnet, insbesondere kann die erste Einheit aber auch direkt an der zweiten Einheit befestigt oder angeordnet sein. Eine erste Einheit, die auf der von einer zweiten Einheit zugewandten Seite einer dritten Einheit angeordnet ist, muss nicht Teil der dritten Einheit sein oder von der dritten Einheit umfasst werden. Eine erste Einheit ist in diesem Fall insbesondere von der dritten Einheit aus gesehen vor der zweiten Einheit angeordnet, insbesondere kann die erste Einheit aber auch direkt an der zweiten Einheit befestigt oder angeordnet sein.

Im Folgenden wird die Erfindung anhand der in den Figuren dargestellten Ausführungsbeispiele näher beschrieben und erläutert.

Es zeigen:
- Fig. 1: eine perspektivische Ansicht eines medizinischen Bildgebungssystems,
- Fig. 2: eine Magneteinheit mit einem inneren und einem äußeren Fenster,
- Fig. 3: eine Magneteinheit mit einem Trichter,
- Fig. 4: einen Schnitt durch das medizinische Bildgebungssystem senkrecht zur Untersuchungsachse,
- Fig. 5: einen Schnitt durch das medizinische Bildgebungssystem senkrecht zur Untersuchungsachse, wobei die Magneteinheit gedreht wurde,
- Fig. 6: einen Schnitt durch das medizinische Bildgebungssystem parallel zur Untersuchungsachse,
- Fig. 7: einen Schnitt durch den Trichter einer Magneteinheit,
- Fig. 8: einen Schnitt durch die Fenster einer Magneteinheit,
- Fig. 9: ein medizinische Bildgebungssystem mit einer zweiten Röntgenquelle und einem zweiten Röntgendetektor,
- Fig. 10: ein medizinisches Bildgebungssystem mit einer ersten Teilchenstrahlungseinheit umfassend einen Teilchenbeschleuniger und eine Teilchenstrahlführung,
- Fig. 11: ein medizinisches Bildgebungssystem mit einer Teilchenstrahlungseinheit, wobei die Teilchenstrahlungseinheit elektrisch geladenen Teilchenstrahlung sendet.

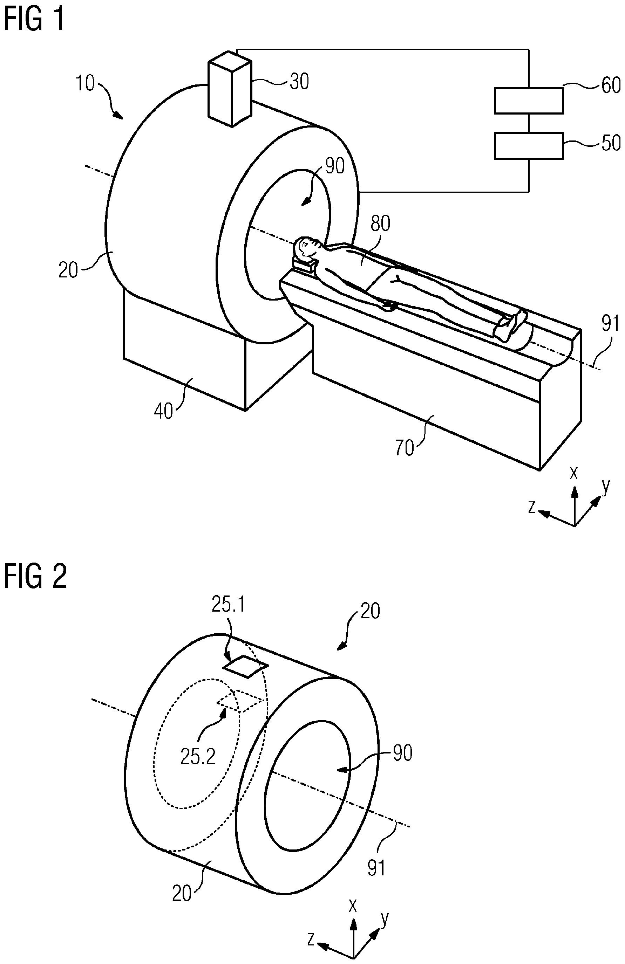

Fig. 1 zeigt eine perspektivische Ansicht eines medizinischen Bildgebungssystems 10. In diesem Ausführungsbeispiel umfasst das medizinischen Bildgebungssystem 10 eine Magneteinheit 20, eine erste Röntgenquelle 30, eine Lagerungs- und Rotationsvorrichtung 40, eine MR-Steuer- und Auswerteeinheit 50, eine Röntgensteuer- und Auswerteeinheit 60 sowie eine Lagerungsvorrichtung 70, auf der sich ein Untersuchungsobjekt 80 befindet. Die Magneteinheit 20 bildet eine Untersuchungsöffnung 90 aus, der zur Aufnahme der Patientenlagerungsvorrichtung 70 mit dem Patienten 80 ausgebildet ist.

In diesem Ausführungsbeispiel ist die Magneteinheit 20 hohlzylinderförmig um eine Untersuchungsachse 91 ausgebildet, wobei die Untersuchungsachse 91 parallel zu einer dritten Koordinatenachse z verläuft. Weiterhin sind eine erste Koordinatenachse x und eine zweite Koordinatenachse y abgebildet, die zusammen mit der dritten Koordinatenachse z ein dreidimensionales karthesisches Koordinatensystem bilden.

In diesem Ausführungsbeispiel ist die Magneteinheit 20 mittels der Lagerungs- und Rotationsvorrichtung 40 rotierbar um die Untersuchungsöffnung 90, insbesondere rotierbar um die Untersuchungsachse 91 ausgebildet. Gleichzeitig ist die erste Röntgenquelle 30 fest mit der Magneteinheit 20 verbunden, so dass bei einer Rotation der Magneteinheit 20 um die Untersuchungsachse 91 auch die erste Röntgenquelle 30 um die Untersuchungsachse 91 sowie um die Untersuchungsöffnung 90 rotiert wird.

Die Magneteinheit 20 ist mit der MR-Steuer- und Auswerteeinheit 50 verbunden. Die erste Strahlungseinheit 30 ist mit der Strahlungssteuer- und Auswerteeinheit 60 verbunden. Weiterhin ist die MR-Steuer- und Auswerteeinheit 50 mit der Strahlungssteuer- und Auswerteeinheit 60 verbunden, insbesondere können die MR-Steuer- und Auswerteeinheit 50 und die Strahlungssteuer- und Auswerteeinheit 60 untereinander Bildinformationen und/oder Steuersignale austauschen. Es ist auch alternativ möglich, dass die MR-Steuer- und Auswerteeinheit 50 und die Strahlungssteuer- und Auswerteeinheit 60 in einer gemeinsamen Steuer- und Auswerteeinheit ausgeführt sind.

Im dargestellten Ausführungsbeispiel ist das Untersuchungsobjekt 80 ein Patient 80, und die Lagerungsvorrichtung 70 eine Patientenlagerungsvorrichtung 70. Die Patientenlagerungsvorrichtung 70 ist dazu ausgebildet, den Patienten 80 in die zylindrisch ausgebildete Untersuchungsöffnung 90 zu transportieren.

Fig. 2 zeigt eine Magneteinheit 20 mit einem äußeren Fenster 25.1 und einem inneren Fenster 25.2, wobei die Fenster als Teil des Gehäuses 26 der Magneteinheit 20 ausgebildet sind. In diesem Ausführungsbeispiels ist der erste Bereich der Magneteinheit 20 durch das äußere Fenster 25.1 und das innere Fenster 25.2 begrenzt. Das äußere Fenster 25.1 und das innere Fenster 25.2 sind hier aus Glas ausgebildet. Die beiden Fenster 25.1, 25.2 können aber auch aus Beryllium, Aluminium oder einem anderen Material ausgebildet sein, wobei das andere Material transparent für die Strahlung 32 der ersten Strahlungseinheit 30 ist. Im gezeigten Ausführungsbeispiel ist das äußere Fenster 25.1 und das innere Fenster 25.2 rechteckig ausgebildet. Die beiden Fenster 25.1, 25.2 können aber auch rund, oval oder in einer anderen Form ausgebildet sein. Bei der Gestaltung des äußeren Fensters 25.1 und des inneren Fensters 25.2 können insbesondere die notwendige Stabilität der Magneteinheit 20 und die geometrische Form des Strahlengangs der Strahlung 32 der ersten Strahlungseinheit 30 berücksichtigt werden.

In dem in Fig. 2 gezeigten Ausführungsbeispiel ist die Magneteinheit 20 zum Rotieren um die Untersuchungsachse 91 ausgebildet und nur genau ein erster Bereich der Magneteinheit 20 durch das äußere Fenster 25.1 und das innere Fenster 25.2 begrenzt. Falls die Magneteinheit 20 nicht zum Rotieren um die Untersuchungsachse 91 ausgebildet ist, ist es alternativ möglich, eine Magneteinheit 20 mit mehreren ersten Bereichen und damit mehreren äußere Fenster 25.1 und innere Fenstern 25.2 zu verwenden, oder eine Magneteinheit 20 mit einem größeren ersten Bereich definiert durch ein größeres äußeres Fenster 25.1 und durch ein größeres inneres Fenster 25.2 zu verwenden, um mit der ersten Strahlungseinheit 30 aus verschiedenen Richtungen auf das Untersuchungsobjekt 80 zu strahlen. Im gezeigten Ausführungsbeispiel sind das äußere Fenster 25.1 und das innere Fenster 25.2 bogenförmig an die Krümmung des Gehäuses 26 der Magneteinheit 20 angepasst. Es ist auch möglich, ein flaches äußeres Fenster 25.1 und/oder ein flaches inneres Fenster 25.2 zu verwenden.

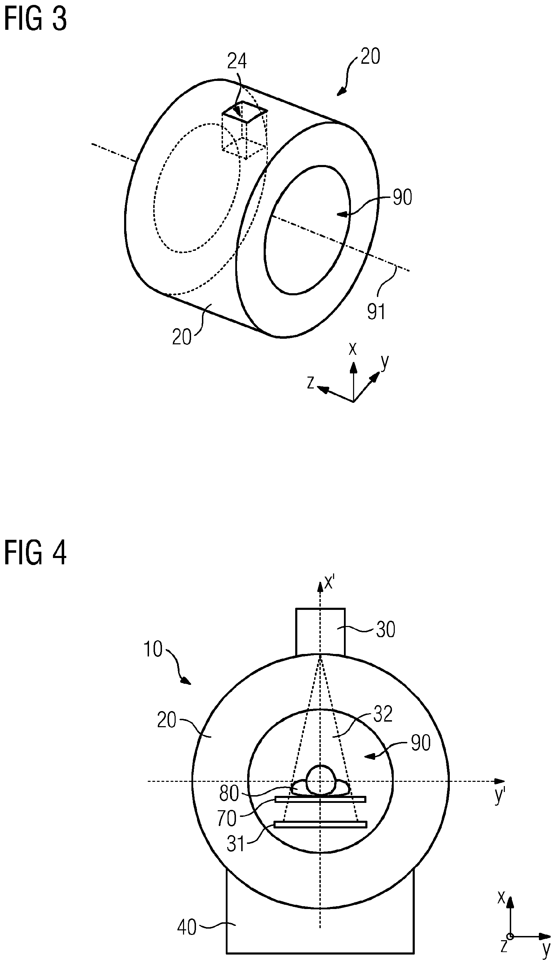

Fig. 3 zeigt eine Magneteinheit 20, die als ersten Bereich einen Trichter 24 ausbildet. Der Trichter 24 wird insbesondere durch das Gehäuse 26 der Magneteinheit 20 ausgebildet. Der Trichter 24 ist in diesem Ausführungsbeispiel als Prisma mit rechteckiger Grundfläche ausgebildet. Der Trichter 24 kann aber auch als Prisma mit einer andersförmigen Grundfläche ausgebildet sein, oder als Pyramidenstumpf oder als Kegelstumpf. Insbesondere ist es möglich, dass der Trichter 24 zylinderförmig ausgebildet ist. Bei der Gestaltung des Trichters 24 können insbesondere die notwendige Stabilität der Magneteinheit 20 und die geometrische Form des Strahlengangs der Strahlung 32 der ersten Strahlungseinheit 30 berücksichtigt werden. Im dargestellten Ausführungsbeispiel ist der Trichter 24 mit Luft gefüllt. Es ist aber auch möglich, den Trichter 24 mit einem anderen Material zu füllen, das von der von der ersten Strahlungseinheit 30 ausgesendeten Strahlung 32 durchdrungen werden kann. Wenn es sich bei der ersten Strahlungseinheit 30 um eine erste Röntgenquelle 30 handelt, kann der Trichter 24 insbesondere auch mit Plexiglas ausgefüllt sein.

In dem in Fig. 3 gezeigten Ausführungsbeispiel ist die Magneteinheit 20 zum Rotieren um die Untersuchungsachse 91 ausgebildet und nur genau ein erster Bereich der Magneteinheit 20 durch einen Trichter 24 ausgebildet. Falls die Magneteinheit 20 nicht zum Rotieren um die Untersuchungsachse 91 ausgebildet ist, ist es alternativ möglich, eine Magneteinheit 20 mit mehreren ersten Bereichen und damit mehrere Trichter 24 zu verwenden, oder eine Magneteinheit 20 mit einem größeren ersten Bereich mit einem größeren Trichter 24 zu verwenden, um mit der ersten Strahlungseinheit 30 aus verschiedenen Richtungen auf das Untersuchungsobjekt 80 zu strahlen.

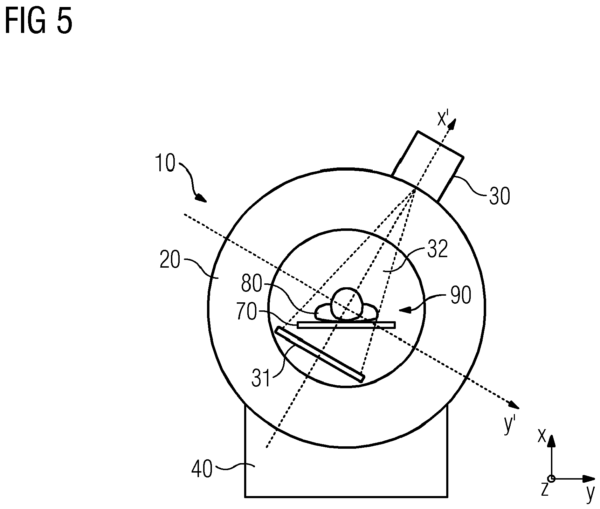

Fig. 4 zeigt einen Schnitt durch das medizinische Bildgebungssystem 10 orthogonal zur Untersuchungsachse 91, insbesondere einen Schnitt durch die Magneteinheit 20. Fig. 5 zeigt ebenfalls einen Schnitt durch das medizinische Bildgebungssystem 10 orthogonal zur Untersuchungsachse 91, insbesondere einen Schnitt durch die Magneteinheit 20. Im gezeigten Ausführungsbeispiel ist die Magneteinheit 20 zum Rotieren ausgebildet, weiterhin ist die erste Strahlungseinheit 30 fest mit der Magneteinheit 20 verbunden und dazu ausgebildet, gemeinsam und simultan mit der Magneteinheit 20 um die Untersuchungsöffnung 90 zu rotieren. Die Orientierung der Magneteinheit 20 ist durch eine erste gedrehte Koordinatenachse x' und eine zweite gedrehte Koordinatenachse y' gegeben, wobei die zweite gedrehte Koordinatenachse y' orthogonal zur ersten gedrehten Koordinatenachse x' ist, und wobei der erste gedrehte Koordinatenachse x' und die zweite gedrehte Koordinatenachse y' orthogonal zur Untersuchungsachse 91 und damit zur dritten Koordinatenachse z sind. Im gezeigten Ausführungsbeispiel entspricht die erste Strahlungseinheit 30 einer ersten Röntgenquelle, weiterhin umfasst das medizinische Bildgebungssystem 10 einen ersten Röntgendetektor 31. Es ist natürlich auch möglich, dass die Magneteinheit 20 mit einer anderen ersten Strahlungseinheit 30 verbunden und gleichzeitig zum Rotieren um die Untersuchungsachse 91 ausgebildet ist. Im gezeigten Ausführungsbeispiel der Fig. 4 und der Fig. 5 ist der erste Röntgendetektor 31 dazu ausgebildet, simultan mit der ersten Röntgenquelle 30 und damit simultan mit der Magneteinheit 20 zu rotieren. Alternativ könnte der erste Röntgendetektor 31 auch ortsfest ausgebildet sein, in diesem Fall muss ein deutlich größerer und/oder ein gekrümmter erster Röntgendetektor 31 verwendet werden, um die Röntgenstrahlung 32 der ersten Röntgenquelle 30 aus verschiedenen Richtungen zu detektieren.

Im gezeigten Ausführungsbeispiel handelt es sich bei der ersten Strahlungseinheit 30 um eine erste Röntgenquelle 30, die dazu ausgebildet ist, Röntgenstrahlung 32 zu senden. Die erste Strahlungseinheit 30 kann aber auch dazu ausgebildet sein, Gammastrahlung oder Teilchenstrahlung zu senden.

Im gezeigten Ausführungsbeispiel ist die erste Röntgenquelle 30 als erste Röntgenröhre 30 mit einer rotierenden Anode (ein englischer Fachbegriff ist "rotating anode") ausgebildet. Eine erste Röntgenröhre 30 kann insbesondere zusammen mit einem ersten Röntgendetektor 31 zur Röntgenbildgebung des Untersuchungsobjekts 80 ausgebildet sein, indem Röntgenprojektionen des Untersuchungsobjekts 80 aufgenommen werden. Dabei sind eine erste Röntgenröhre mit rotierender Anode und ein erster Röntgendetektor 31 aus dem Stand der Technik bekannt. Unter Verwendung mehrerer Röntgenprojektionen eines Untersuchungsobjekts bezüglich verschiedener Projektionsrichtungen kann auch ein dreidimensionaler Röntgenbilddatensatz rekonstruiert werden.

Eine erste Röntgenquelle 30 kann alternativ auch als erste Röntgenröhre mit statischer Anode (ein englischer Fachbegriff ist "static anode") oder mit einer Anode bestehend aus einem flüssigen Metallstrahl (ein englischer Fachbegriff ist "liquid metal jet anode"). Weiterhin kann eine erste Röntgenquelle als Linearbeschleuniger ausgebildet sein (ein englischer Fachbegriff ist "linear accelerator", kurz LINAC). Im Vergleich mit einer Röntgenröhre kann mit einem Linearbeschleuniger insbesondere Röntgenstrahlung mit einer kleineren Wellenlänge erzeugt werden. Diese Röntgenstrahlung kann dann insbesondere für die Manipulation eines Bereichs des Untersuchungsobjekts 80 verwendet werden. Dabei kann insbesondere durch die Bestrahlung Gewebe zerstört werden, insbesondere Tumorgewebe. Mit einem Linearbeschleuniger können auch Teilchenstrahlung erzeugt werden.

Ein Linearbeschleuniger weist eine entlang einer Achse ausgebildete lineare Beschleunigungseinheit auf. Die lineare Beschleunigungseinheit kann parallel zur ersten gedrehten Koordinatenachse x' ausgebildet sein. Die lineare Beschleunigungseinheit kann auch in eine andere Richtung ausgebildet sein, insbesondere parallel zur dritten Koordinatenachse z oder parallel zur zweiten gedrehten Koordinatenachse y'. In diesem Fall ist der Platzbedarf oberhalb der Magneteinheit 20 besonders klein, und das medizinische Bildgebungssystem 10 kann in standardisierten Untersuchungsräumen eingesetzt werden, es muss aber bei Teilchenstrahlung dann eine zusätzliche Umlenkeinheit verwendet werden, um die Strahlung 32 korrekt durch den ersten Bereich der Magneteinheit 20 zu senden.

Ein erster Röntgendetektor 31 kann wie im dargestellten Ausführungsbeispiel flächig ausgebildet sein. Hierbei sind für einen flachen Röntgendetektor 31 verschiedene Ausführungsformen bekannt, beispielsweise bestehend aus amorphem Silicium oder bestehend aus sich ergänzenden Metall-Oxid-Halbleitern (ein englischer Fachbegriff ist "complementary metal-oxidsemiconductor", eine gebräuchliche Abkürzung ist "CMOS"). Weiterhin sind photonenzählende erste Röntgendetektoren sowie erste Röntgendetektoren umfassend einen Schirm bekannt (ein englischer Fachbegriff ist "screen-film"), wobei der Schirm Röntgenstrahlung 32 in sichtbares Licht umwandelt. Der erste Röntgendetektor 31 kann alternativ auch gekrümmt oder stückweise gekrümmt ausgebildet sein.

Der erste Röntgendetektor 31 kann wie im gezeigten Ausführungsbeispiel der Fig. 4 und Fig. 5 innerhalb der Untersuchungsöffnung 90 ausgebildet sein. Weiterhin kann der Röntgendetektor auch zwischen dem Hauptmagneten 21 und der Untersuchungsöffnung 90 ausgebildet sein, insbesondere zwischen den Teilen oder innerhalb der Gradientenspuleneinheit 22 oder den Teilen oder innerhalb der Hochfrequenzantenneneinheit 23. Alternativ kann der erste Röntgendetektor 31 aber auch außerhalb der Magneteinheit 20 auf der von der ersten Röntgenquelle abgewandten Seite der Magneteinheit 20 angeordnet sein. In diesem Fall weist die Magneteinheit 20 einen für die Strahlung 32 der ersten Strahlungseinheit 30 transparenten Austrittsbereich 27 auf, wobei die Strahlung 32 nach dem Passieren des Untersuchungsobjekts 80 durch den Austrittsbereich 27 verläuft.

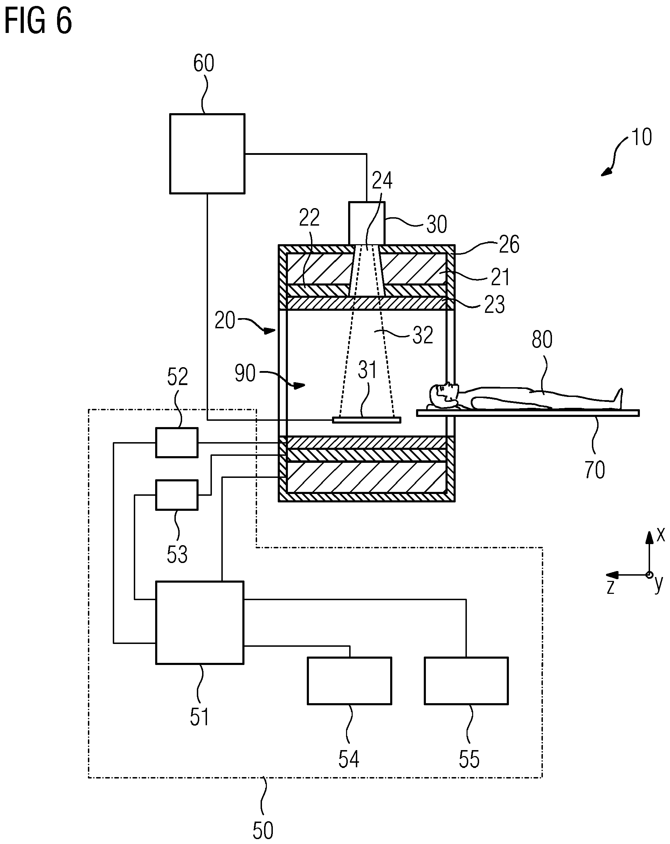

In Fig. 6 ist die Funktionsweise einer Magneteinheit 20 schematisch anhand eines Schnitts orthogonal zur zweiten Koordinatenachse y dargestellt. Die Magneteinheit 20 umschließt eine Untersuchungsöffnung 90 zu einer Aufnahme eines Patienten 80. Die Untersuchungsöffnung 90 im vorliegenden Ausführungsbeispiel ist zylinderförmig ausgebildet und in einer Umfangsrichtung von der Magneteinheit 20 hohlzylinderförmig umgeben. Grundsätzlich ist jedoch eine davon abweichende Ausbildung der Untersuchungsöffnung 90 jederzeit denkbar. Der Patient 80 kann mittels einer Patientenlagerungsvorrichtung 70 des in die Untersuchungsöffnung 90 geschoben werden. Die Patientenlagerungsvorrichtung 70 weist hierzu einen innerhalb der Untersuchungsöffnung 90 bewegbar ausgestalteten Patiententisch auf. Die Magneteinheit 20 umfasst einen Hauptmagneten 21 zu einem Erzeugen eines starken und insbesondere homogenen Hauptmagnetfelds innerhalb der Untersuchungsöffnung 90. Die Magneteinheit 20 ist mittels eines Gehäuses 26 nach außen abgeschirmt.

Die Magneteinheit 20 weist weiterhin eine Gradientenspuleneinheit 22 zu einer Erzeugung von Magnetfeldgradienten auf, die für eine Ortskodierung während einer Bildgebung verwendet werden. Die Gradientenspuleneinheit 22 wird mittels einer Gradientensteuereinheit 53 der MR-Steuer- und Auswerteeinheit 50 gesteuert. Die Magneteinheit 20 umfasst weiterhin eine Hochfrequenzantenneneinheit 23, welche im vorliegenden Ausführungsbeispiel als fest in die Magneteinheit 20 integrierte Körperspule ausgebildet ist. Die Hochfrequenzantenneneinheit 23 ist zu einer Anregung von Atomkernen, die sich in dem von dem Hauptmagneten 21 erzeugten Hauptmagnetfeld einstellt, ausgelegt. Die Hochfrequenzantenneneinheit 23 wird von einer Hochfrequenzantennensteuereinheit 52 der MR-Steuer- und Auswerteeinheit 50 gesteuert und strahlt hochfrequente Wechselfelder in einen Untersuchungsraum ein, der im Wesentlichen von einer Untersuchungsöffnung 90 der Magneteinheit 20 gebildet ist. Die Hochfrequenzantenneneinheit 23 ist weiterhin zum Empfang von Magnetresonanzsignalen ausgebildet.

Die Gradientenspuleneinheit 22 kann insbesondere Magnetfelder mit einem Gradienten in Richtung der ersten gedrehten Koordinatenachse x', in Richtung der zweiten gedrehten Koordinatenachse y' oder in Richtung der dritten Koordinatenachse y erzeugen. Die Gradientenspuleneinheit 22 umfasst hierfür in diesem Ausführungsbeispiel drei Gradientenspulenteileinheiten, die jeweils ein Magnetfeld mit einem Gradienten in Richtung einer der Koordinatenachsen x', y', z' erzeugen können. Für jede der drei Gradientenspulenteileinheiten ist hierbei eine Anordnung bekannt, so dass jede Gradientenspulenteileinheit außerhalb des ersten Bereichs der Magneteinheit 20 angeordnet ist.

Zu einer Steuerung des Hauptmagneten 21, der Gradientenspuleneinheit 22 und zur Steuerung der Hochfrequenzantenneneinheit 23 ist die Magneteinheit 20 mit einer MR-Steuer- und Auswerteeinheit 50 verbunden. Die MR-Steuer- und Auswerteeinheit 50 steuert zentral mittels einer Systemsteuereinheit 51 die Magneteinheit 20, wie beispielsweise das Durchführen einer vorbestimmten bildgebenden Gradientenechosequenz. Dabei erfolgt die Steuerung über eine Hochfrequenzantennensteuereinheit 52 und eine Gradientensteuereinheit 53. Zudem umfasst die MR-Steuer- und Auswerteeinheit 50 eine nicht näher dargestellte Auswerteeinheit zu einer Auswertung von medizinischen Bilddaten, die während der Magnetresonanzuntersuchung erfasst werden. Des Weiteren umfasst die MR-Steuer- und Auswerteeinheit 50 eine nicht näher dargestellte Benutzerschnittstelle, diese umfasst eine Anzeigeeinheit 54 und eine Eingabeeinheit 55, die jeweils mit der Systemsteuereinheit 51 verbunden sind. Steuerinformationen wie beispielsweise Bildgebungsparameter, sowie rekonstruierte Magnetresonanzbilder können auf der Anzeigeeinheit 54, beispielsweise auf zumindest einem Monitor, für ein medizinisches Bedienpersonal angezeigt werden. Mittels der Eingabeeinheit 55 können Informationen und/oder Parameter während eines Messvorgangs von dem medizinischen Bedienpersonal eingegeben werde.

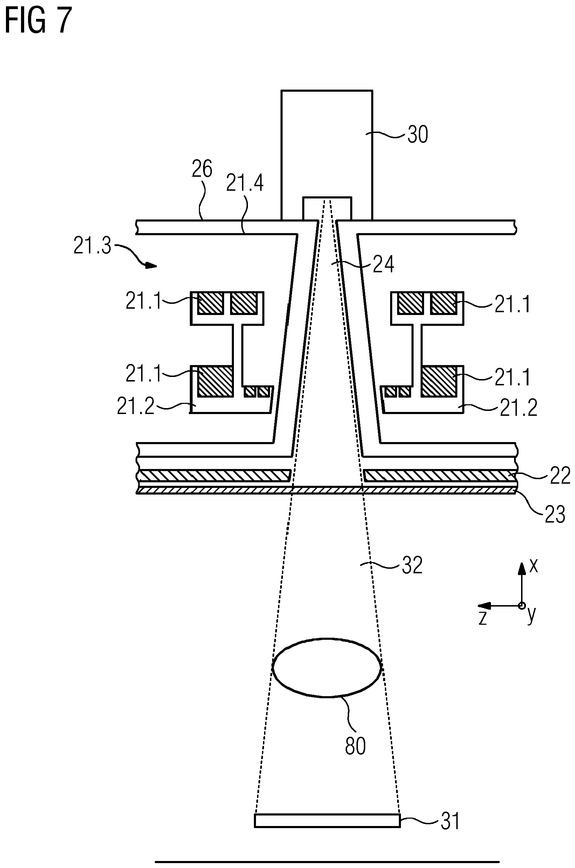

Fig. 7 zeigt einen Schnitt orthogonal zur ersten gedrehten Koordinatenachse y' durch einen Trichter 24 durch die Magneteinheit 20. Im gezeigten Ausführungsbeispiel verläuft der Trichter 24 durch den Hauptmagneten 21 und die Gradientenspuleneinheit 22, aber nicht durch die Hochfrequenzantenneneinheit 23. In diesem Ausführungsbeispiel ist die Hochfrequenzantenneneinheit 23 zumindest teilweise für Strahlung 32 der ersten Strahlungseinheit 30 durchdringbar ausgebildet. Es ist auch möglich, dass der Trichter 24 die Hochfrequenzantenneneinheit 23 durchdringt. Weiterhin ist es ebenfalls möglich, dass der Trichter 24 die Gradientenspuleneinheit 22 nicht durchdringt, in diesem Fall muss die Gradientenspuleneinheit 22 zumindest teilweise für Strahlung 32 der ersten Strahlungseinheit 30 durchdringbar ausgebildet sein. Der Trichter 24 durchdringt den Hauptmagneten 21 derart, dass der Hauptmagnet 21, insbesondere die Spulenelemente 21.1, ein homogenes Magnetfeld in der Untersuchungsöffnung 90 erzeugen kann. Im gezeigten Ausführungsbeispiel umfasst der Hauptmagnet 21 mehrere supraleitende Spulenelemente 21.1 auf einem Spulenträger 21.2, die von einem Kühlmittel 21.3 umgeben sind. Die supraleitenden Spulenelemente 21.1 umlaufen hierbei die Untersuchungsöffnung 90. Der Spulenträger 21.2 sorgt einerseits für die mechanische Stabilität und die Formstabilität der Spulenelemente 21.1, als auch für die Kühlung der Spulenelemente 21.1.

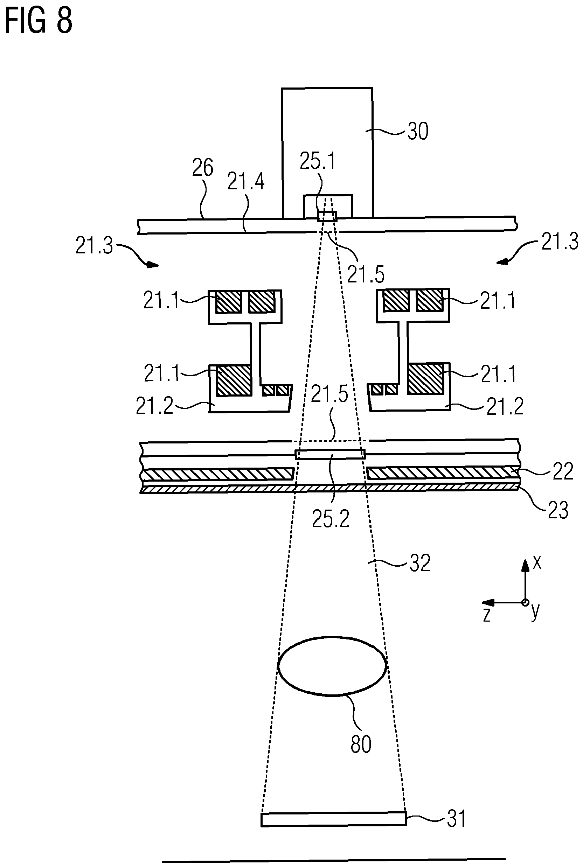

Fig. 8 zeigt einen Schnitt orthogonal zur ersten gedrehten Koordinatenachse y' durch das äußere Fenster 25.1 und das innere Fenster 25.2 der Magneteinheit 20. Das äußere Fenster 25.1 und das innere Fenster 25.2 sind im gezeigten Ausführungsbeispiel ein Teil des Gehäuses 26 der Magneteinheit 20. Sowohl das äußere Fenster 25.1 als auch das innere Fenster 25.2 sind hier rechteckig ausgebildet. Es sind natürlich auch andere Fensterformen möglich, insbesondere runde Fenster 25.1, 25.2. In diesem Ausführungsbeispiel weist die thermische Isolierung 21.4 ebenfalls Bereiche 21.5 auf, die für die Strahlung 32 der ersten Strahlungseinheit 30 durchdringbar sind. Diese Bereiche 21.5 der thermischen Isolierung 21.4 können beispielsweise aus dem Material der thermischen Isolierung 21.4 aufgebaut sein, jedoch dünner ausgebildet sein als das Gehäuse 26 außerhalb des Bereichs. Die Bereiche 21.5 der thermischen Isolierung 21.4 können auch aus einer Metallfolie ausgebildet sein, insbesondere aus einer Aluminiumfolie oder einer Kupferfolie.

Das äußere Fenster 25.1 und das innere Fenster 25.2 bestehen im gezeigten Ausführungsbeispiel aus Beryllium. Es ist weiterhin möglich, die Fenster 25.1, 25.2 aus einem anderen Material auszubilden, dass für die Strahlung 32 der ersten Strahlungseinheit 30 transparent ist, beispielsweise aus Aluminium oder Glas.

Im gezeigten Ausführungsbeispiel ist das elektrisch leitende Material der Spulenelemente 21.1 Magnesiumdiborid MgB₂. Die kritische Temperatur 39 K von Magnesiumdiborid MgB₂ liegt über der Siedetemperatur 4.2 K von Helium bei Normaldruck 1013 hPa. Es sind auch alternative elektrisch leitende Materialien für die Spulenelemente 21.1 vorstellbar, insbesondere supraleitende Materialien, und insbesondere supraleitende Materialien mit einer kritischen Temperatur über der Siedetemperatur 4.2 K von Helium bei Normaldruck 1013 hPa, beispielsweise Niobgermanium Nb₃Ge mit einer kritischen Temperatur von 23 K.

Sowohl in dem in Fig. 7 als auch in dem in Fig. 8 dargestellten Ausführungsbeispiel erfolgt die Kühlung durch Wärmeleitung über den Spulenträger 21.2, indem der Spulenträger 21.2 mittels eines Röhrensystems gekühlt wird, wobei im Röhrensystem ein Kühlmittel zum Wärmetransport zirkuliert und die Wärme zu einem Wärmetauscher transportiert. Dabei kann unterstützend mittels eines optionalen gasförmigen Kühlmittels 21.3 gekühlt werden. Das in der Fig. 7 nicht dargestellte Röhrensystem ist derart ausgebildet, dass es nicht im ersten Bereich der Magneteinheit 20 ausgebildet ist, um die Transparenz des ersten Bereichs für Strahlung 32 der ersten Strahlungseinheit 30 zu verbessern. Alternativ und insbesondere bei nicht zum Rotieren ausgelegten Magneteinheiten 20 kann die Kühlung der Spulenelemente 21.1 durch Immersion in ein Kühlmittel 21.3, beispielsweise in flüssiges Helium 21.3 erfolgen. Ein derartiges Röhrensystem ist beispielsweise aus der Druckschrift DE 10 2004 061 869 B4 bekannt.

Sowohl in dem in Fig. 7 als auch in dem in Fig. 8 dargestellten Ausführungsbeispiel sind die Spulenelemente 21.1 des Hauptmagneten 21 und der Spulenträger 21.2 des Hauptmagneten 21 nicht im ersten Bereich der Magneteinheit 20 angeordnet. Auch sind keine Teile der Spulenelemente 21.1 und keine Teile des Spulenträgers 21.2 im ersten Bereich der Magneteinheit 20 angeordnet. Bei einer Magneteinheit 20 umlaufen die Spulenelemente 21.1 üblicherweise ringförmig die Untersuchungsachse 91, um bei Stromdurchfluss in der Untersuchungsöffnung 90 ein homogenes Magnetfeld in Richtung der dritten Koordinatenachse z zu erzeugen. Die ringförmigen Spulenelemente 21.1 weisen zueinander einen Abstand bezüglich der dritten Koordinatenachse z auf. Damit keine Teile der Spulenelemente 21.1 im ersten Bereich der Magneteinheit 20 ausgebildet sind, kann zwischen zwei verschiedenen Spulenelementen 21.1 insbesondere ein Abstand gewählt werden, der größer ist als die Ausdehnung des ersten Bereichs bezüglich der dritten Koordinatenachse Z, und die zwei verschiedenen Spulenelemente 21.1 können auf unterschiedlichen Seiten des ersten Bereichs angeordnet werden. Dieser Abstand kann insbesondere größer sein als die Abstände zwischen allen anderen benachbarten Spulenelementen 21.1. In dem in Fig. 7 als auch in dem in Fig. 8 dargestellten Ausführungsbeispiel bildet der Spulenträger 21.2 im ersten Bereich eine durchgehende Öffnung aus, die größer ist als die Ausdehnung des Strahlengangs der Strahlung 32 durch den Spulenträger 21.2. Alternativ ist es denkbar, auf unterschiedlichen Seiten des ersten Bereichs jeweils mindestens einen separaten Spulenträger 21.2 anzuordnen. Weiterhin ist es auch möglich, als Material des Spulenträgers 21.2 ein Material zu verwenden, das für die Strahlung 32 der ersten Strahlungseinheit 30 transparent ist. Der Spulenträger 21.2 besteht vorteilhafterweise aus einem Material mit hoher Wärmeleitfähigkeit und niedriger mechanischer Verformbarkeit, insbesondere aus Metall, insbesondere aus Aluminium.

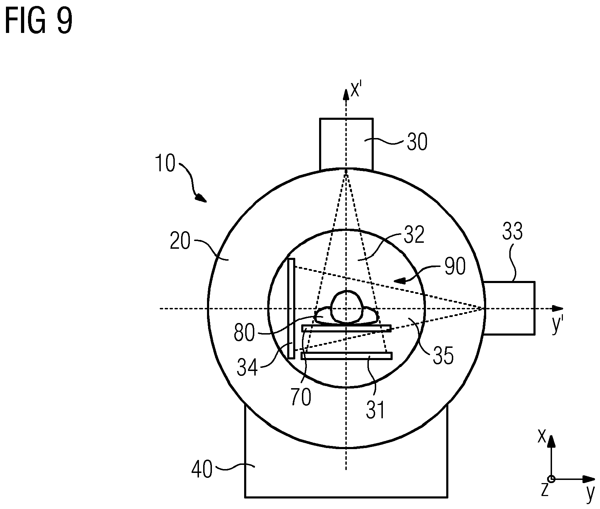

Fig. 9 zeigt einen schematischen Schnitt durch ein medizinisches Bildgebungssystem 10 mit einer ersten Röntgenquelle 30, einer zweiten Röntgenquelle 33, einem ersten Röntgendetektor 31 und einem zweiten Röntgendetektor 34. Hierbei ist die erste Röntgenquelle 30 dazu ausgebildet, Röntgenstrahlung 32 durch die Magneteinheit 20 und durch das Untersuchungsobjekts 80 in Richtung der ersten gedrehten Koordinatenachse x' zum ersten Röntgendetektor 31 auszusenden, die zweite Röntgenquelle 33 ist dazu ausgebildet, Röntgenstrahlung 35 durch die Magneteinheit 20 und durch das Untersuchungsobjekts 80 in Richtung der zweiten gedrehten Koordinatenachse y' zum zweiten Röntgendetektors 34 auszusenden. Dabei ist die gedrehte Koordinatenachse x' orthogonal zur gedrehten Koordinatenachse y', und beide gedrehte Koordinatenachsen sind orthogonal zur Untersuchungsachse 91 und damit zur Koordinatenachse z. Durch diese Anordnung können gleichzeitig zwei Röntgenprojektionen bezüglich orthogonaler Projektionsrichtungen aufgenommen werden. Mit den zwei Röntgenprojektionen kann ein dreidimensionaler Bilddatensatz rekonstruiert werden. Im gezeigten Ausführungsbeispiel ist die Magneteinheit 20 dazu ausgebildet, um die Untersuchungsöffnung 90, insbesondere um die Untersuchungsachse 91 zu rotieren. Die erste Röntgenquelle 30, der erste Röntgendetektor 31, die zweite Röntgenquelle 33 und der zweite Röntgendetektor 34 sind dazu ausgebildet, simultan mit der Magneteinheit 20 um die Untersuchungsöffnung 90, insbesondere um die Untersuchungsachse 91 zu rotieren, beispielsweise indem sie fest mit der Magneteinheit 20 verbunden sind.

Die Verbindungslinie zwischen der ersten Röntgenquelle 30 und dem ersten Röntgendetektor 31 entspricht in der Fig. 9 der Koordinatenachse x', die Verbindungslinie zwischen der zweiten Röntgenquelle 33 und dem zweiten Röntgendetektor 34 entspricht der Koordinatenachse y'. In diesem Ausführungsbeispiel sind die Verbindungslinien zueinander orthogonal, es sind aber auch andere Winkel möglich.

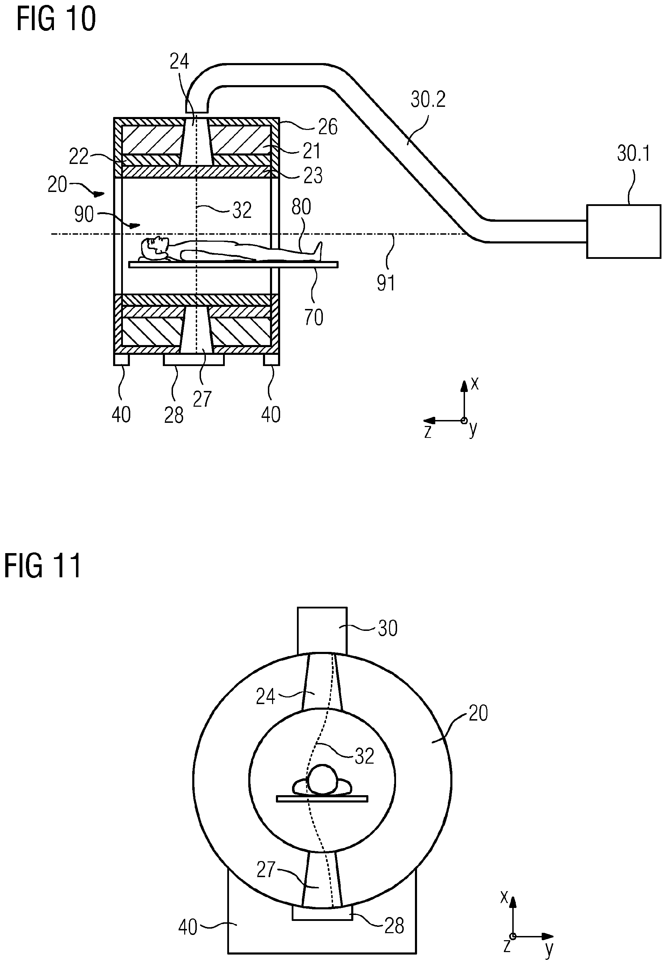

Fig. 10 zeigt einen schematischen Schnitt durch ein medizinisches Bildgebungssystem 10 orthogonal zur zweiten Koordinatenachse y. Das medizinische Bildgebungssystem 10 umfasst neben der Magneteinheit 20 eine Strahlungseinheit 30, die einen Teilchenbeschleuniger 30.1 und einer bewegliche Teilchenstrahlführung 30.2 für geladene Teilchen umfasst (ein englischer Fachbegriff für eine bewegliche Teilchenstrahlführung ist "gantry"). In diesem Ausführungsbeispiel ist der Teilchenbeschleuniger 30.1 ortsfest ausgebildet, und die Teilchenstrahlführung 30.2 ist dazu ausgebildet, um die Untersuchungsöffnung 90, insbesondere um die Untersuchungsachse 91 zu rotieren. Weiterhin ist im gezeigten Ausführungsbeispiel die Magneteinheit 20 dazu ausgebildet, um die Untersuchungsöffnung 90 zu rotieren. Dabei sind die Teilchenstrahlführung 30.2 und die Magneteinheit 20 zum simultanen Rotieren ausgebildet. Die bewegliche Teilchenstrahlführung 30.2 umfasst Umlenkeinheiten, die ein Magnetfeld erzeugen und mittels der auf geladene Teilchen wirkende Lorentzkraft die Teilchen auf Kurven entlang der Teilchenstrahlführung 30.2 leitet. Dabei kann die Stärke der Magnetfelder der Teilchenstrahlführung 30.2 an die Masse, die Ladung und die Geschwindigkeit der geführten Teilchen angepasst werden.

Das medizinische Bildgebungssystem 10 umfasst in diesem Ausführungsbeispiel weiterhin einen Austrittsbereich 27 sowie eine Abschirmung 28. Der Austrittsbereich 27 ist derart ausgebildet, dass die von der Strahlungseinheit 30 durch den Trichter 24 auf das Untersuchungsobjekt 80 gesendeten Strahlung 32 die Magneteinheit 20 durch den Austrittsbereich 27 verlassen. Dadurch kann die Teilchenstrahlung 32 die Magneteinheit 20 nicht beschädigen. Die Abschirmung 28, die vorteilhafterweise aus Blei ausgebildet ist, absorbiert den Teilchenstrahl 32, um eine Gefährdung durch die Teilchenstrahlung 32 zu verhindern.

Im dargestellten Ausführungsbeispiel ist der Austrittsbereich 27 der Magneteinheit 20 bezüglich der Untersuchungsachse 91 dem Trichter 24 der Magneteinheit 20 direkt gegenüber angeordnet. Es ist aber auch möglich, den Austrittsbereich 27 an einer anderen Position auszubilden, so dass die Ablenkung der Strahlung 32 durch das Hauptmagnetfeld des Hauptmagneten 21 berücksichtigt wird.

Fig. 11 zeigt einen weiteren Schnitt des in Fig. 10 dargestellten medizinischen Bildgebungssystems 10. Da der Hauptmagnet 21, die Gradientenspuleneinheit 22 und die Hochfrequenzantenneneinheit 23 zeitlich konstante oder zeitlich variable magnetische oder elektrische Felder erzeugen, wird Strahlung 32 bestehend aus elektrisch geladenen Teilchen durch die Lorentzkraft abgelenkt. Die erste Strahlungseinheit 30 ist in diesem Beispiel dazu ausgebildet, die Geschwindigkeit und die Richtung der elektrisch geladenen Teilchen im Teilchenstrahl 32 derart anzupassen, dass diese unter Berücksichtigung der in der Untersuchungsöffnung 90 herrschenden elektrischen und magnetischen Felder auf einen vordefinierten Teil des Untersuchungsobjekts 80 treffen.

Um die Teilchenstrahlungsquelle 30 mit dem Magnetfeld der Magneteinheit 20 abzustimmen, sind in diesem Ausführungsbeispiel die Strahlungssteuer- und Auswerteeinheit 60 und die MR-Steuer- und Auswerteeinheit 50 verbunden, dabei gibt die MR-Steuer- und Auswerteeinheit 50 Informationen über das Magnetfeld in der Magneteinheit 20 an die Strahlungssteuer- und Auswerteeinheit 60, welche die Geschwindigkeit und die Richtung der Teilchen der Teilchenstrahlung 32 anpasst. Es ist aber auch möglich, die Strahlungssteuer- und Auswerteeinheit 60 und die MR-Steuer- und Auswerteeinheit 50 als eine gemeinsame Steuer- und Auswerteeinheit auszuführen, die sowohl die Magneteinheit 20 als auch die erste Strahlungseinheit 30 steuert und die gewonnenen Daten auswertet.

Weiterhin überträgt im gezeigten Ausführungsbeispiel die MR-Steuer- und Auswerteeinheit 50 MR-Bilddatensätze an die Strahlungssteuer- und Auswerteeinheit 60. Die Strahlungssteuer- und Auswerteeinheit 60 kann dann die Strahlungseinheit 30 derart steuern, dass die Strahlung 32 auf einen vorbestimmten Teil des Untersuchungsobjekts 80 trifft. Eine mögliche Bewegung des vorbestimmten Teils des Untersuchungsobjekts 80 aufgrund von Veränderungen oder Bewegungen des Untersuchungsobjekts 80 kann dabei durch eine Analyse der MR-Bilddatensätze erkannt und mittels der Strahlungssteuer- und Auswerteeinheit 60 ausgeglichen werden.

## Patentansprüche

1. Medizinisches Bildgebungssystem (10), umfassend eine Magneteinheit (20) ausgebildet zur Magnetresonanzbildgebung eines Untersuchungsobjekts (80) sowie eine erste Strahlungseinheit (30) ausgebildet zum Bestrahlen des Untersuchungsobjekts (80),
- wobei die Magneteinheit (20) einen Hauptmagneten (21) sowie ein Gehäuse (26) aufweist, wobei der Hauptmagnet (21) innerhalb des Gehäuses (26) angeordnet ist, und wobei der Hauptmagnet (21) Spulenelemente (21.1) und wenigstens einen Spulenträger (21.2) aufweist,
- wobei die Magneteinheit (20) derart eine Untersuchungsöffnung (90) entlang einer Untersuchungsachse (91) definiert, dass die Magneteinheit (20) die Untersuchungsöffnung (90) umschließt,
- wobei die Magneteinheit (20) einen ersten Bereich aufweist, der für radial zur Untersuchungsachse (91) ausgesendete Strahlung (32) der ersten Strahlungseinheit (30) transparent ist,
- wobei die erste Strahlungseinheit (30) auf der von der Untersuchungsöffnung (90) abgewandten Seite der Magneteinheit (20) angeordnet ist,
- wobei die erste Strahlungseinheit (30) dazu ausgebildet ist, Strahlung (32) durch den ersten Bereich der Magneteinheit (20) in Richtung der Untersuchungsöffnung (90) zu senden,
- und wobei die erste Strahlungseinheit (30) weiterhin dazu ausgebildet ist, um die Untersuchungsöffnung (90) zu rotieren.

2. Medizinisches Bildgebungssystem (10) nach dem vorherigen Anspruch, wobei die Spulenelemente (21.1) des Hauptmagneten (21) und der wenigstens eine Spulenträger (21.2) außerhalb des ersten Bereichs der Magneteinheit (20) angeordnet sind.

3. Medizinisches Bildgebungssystem (10) nach einem der vorherigen Ansprüche, wobei die Magneteinheit (20) im ersten Bereich wenigstens ein inneres Fenster (25.2) und wenigstens ein äußeres Fenster (25.1) im Gehäuse (26) aufweist, wobei das innere Fenster (25.2) und das äußere Fenster (25.1) für die von der ersten Strahlungseinheit (30) ausgesendete Strahlung (32) transparent sind.

4. Medizinisches Bildgebungssystem (10) nach Anspruch 1 oder 2, wobei der erste Bereich als Trichter (24) in der Magneteinheit (20) ausgebildet ist, der sich radial zur Untersuchungsachse (91) erstreckt und der für die von der ersten Strahlungseinheit (30) radial zur Untersuchungsachse (91) ausgesendete Strahlung (32) transparent ist.

5. Medizinisches Bildgebungssystem (10) einem der vorherigen Ansprüche, wobei die erste Strahlungseinheit (30) eine Teilchenstrahlungsquelle ist, die dazu ausgebildet ist, Teilchenstrahlung (32) zu erzeugen.

6. Medizinisches Bildgebungssystem (10) nach einem der Ansprüche 1 bis 3,
- wobei die erste Strahlungseinheit (30) eine erste Röntgenquelle (30) ist,
- wobei das medizinisches Bildgebungssystem (10) weiterhin einen ersten Röntgendetektor (31) aufweist,
- wobei der erste Röntgendetektor (31) auf der von der ersten Röntgenquelle (30) abgewandten Seite des Untersuchungsobjekts (80) angeordnet ist,
- und wobei die erste Röntgenquelle (30) und der erste Röntgendetektor (31) zur Röntgenbildgebung des Untersuchungsobjekts (80) ausgebildet sind.

7. Medizinisches Bildgebungssystem (10) nach Anspruch 6, wobei der erste Röntgendetektor (31) dazu ausgebildet ist, simultan mit der ersten Röntgenquelle (30) um die Untersuchungsöffnung (90) zu rotieren.

8. Medizinisches Bildgebungssystem (10) nach einem der vorherigen Ansprüche, wobei die Magneteinheit (20) dazu ausgebildet ist, um die Untersuchungsöffnung (90) zu rotieren.

9. Medizinisches Bildgebungssystem (10) nach Anspruch 8, wobei die erste Strahlungseinheit (30) und die Magneteinheit (20) dazu ausgebildet sind, simultan um die Untersuchungsöffnung (90) zu rotieren.

10. Medizinisches Bildgebungssystem (10) nach einem der vorherigen Ansprüche, wobei die Magneteinheit (20) dazu ausgelegt ist, die Spulenelemente (21.1) des Hauptmagneten (21) mittels Wärmeleitung zu kühlen.

11. Medizinisches Bildgebungssystem (10) nach Anspruch 10, wobei die Abwärme der Spulenelemente (21.1) des Hauptmagneten (21) durch Röhrenleitungen umfassend ein zirkulierendes Kühlmittel abgeleitet wird.

12. Medizinisches Bildgebungssystem (10) nach einem der vorherigen Ansprüche, wobei die Spulenelemente (21.1) des Hauptmagneten (21) aus elektrisch supraleitendem Material ausgebildet sind, wobei die kritische Temperatur des supraleitenden Materials höher ist als der Siedepunkt von Helium.

13. Medizinisches Bildgebungssystem (10) nach einem der Ansprüche 6 bis 12, weiterhin umfassend eine zweite Strahlungseinheit (33),
- wobei die Magneteinheit (20) einen zweiten Bereich aufweist, der für radial zur Untersuchungsachse (91) ausgesendete Strahlung (35) der zweiten Strahlungseinheit (33) transparent ist,
- wobei die zweite Strahlungseinheit (33) auf der von der Untersuchungsöffnung (90) abgewandten Seite der Magneteinheit (20) angeordnet ist,
- wobei die zweite Strahlungseinheit (33) dazu ausgebildet ist, Strahlung (35) durch den zweiten Bereich der Magneteinheit (20) in Richtung der Untersuchungsöffnung (90) zu senden,
- und wobei die zweite Strahlungseinheit (33) weiterhin dazu ausgebildet ist, um die Untersuchungsöffnung (90) zu rotieren.

14. Medizinisches Bildgebungssystem (10) nach Anspruch 13, weiterhin aufweisend einen zweiten Röntgendetektor (34),
- wobei die zweite Strahlungseinheit (33) eine zweite Röntgenquelle (33) ist,
- wobei der zweite Röntgendetektor (34) auf der von der zweiten Röntgenquelle (33) abgewandten Seite des Untersuchungsobjekts (80) angeordnet ist,
- wobei der zweite Röntgendetektor (34) dazu ausgebildet ist, simultan mit der zweiten Röntgenquelle (33) um die Untersuchungsöffnung (90) zu rotieren,
- und wobei die zweite Röntgenquelle (33) und der zweite Röntgendetektor (34) zur Röntgenbildgebung des Untersuchungsobjekts (80) ausgebildet sind.

15. Medizinisches Bildgebungssystem (10) nach Anspruch 14, wobei die Verbindungslinie von erster Röntgenquelle (30) und erstem Röntgendetektor (31) mit der Verbindungslinie von zweiter Röntgenquelle (33) und zweitem Röntgendetektor (34) einen Winkel zwischen 60 und 120 Grad oder zwischen 80 und 100 Grad oder zwischen 85 und 95 Grad einschließen.
